Europäisches Patentamt

European Patent Office

Office européen des brevets

(11) Publication number: **0 181 342 B1**

## EUROPEAN PATENT SPECIFICATION

(45) Date of publication of patent specification: **28.10.92**  (51) Int. Cl.⁵: **A61K 37/02**, A61K 31/40, A61K 31/195

(21) Application number: **85901284.1**

(22) Date of filing: **01.03.85**

(86) International application number:
**PCT/US85/00330**

(87) International publication number:
**WO 85/03873 (12.09.85 85/20)**

The file contains technical information submitted
after the application was filed and not included in
this specification

(54) **METHOD OF TREATING HYPERTENSION IN VERTEBRATES.**

(30) Priority: **01.03.84 US 585290**

(43) Date of publication of application:
**21.05.86 Bulletin 86/21**

(45) Publication of the grant of the patent:
**28.10.92 Bulletin 92/44**

(84) Designated Contracting States:
**DE FR GB**

(56) References cited:
| | |
|---|---|
| **LU-A- 67 165** | **US-A- 3 873 296** |
| **US-A- 4 167 564** | **US-A- 4 218 474** |
| **US-A- 4 329 356** | **US-A- 4 340 592** |
| **US-A- 4 374 829** | **US-A- 4 435 424** |

**J. Med. Chem., vol. 25,1982, Safdy, Trypto-
phan Analogues.I. Syntesis and antihyper-
tensive activity of positional isomers, pages
723-730**

(73) Proprietor: **ERK, Vernon
Cedar Hill Associates, Ste. 2860 420 Lexing-
ton Ave.
New York, NY 10170(US)**

(72) Inventor: **ERK, Vernon
Cedar Hill Associates, Ste. 2860 420 Lexing-
ton Ave.
New York, NY 10170(US)**

(74) Representative: **Perry, Robert Edward et al
GILL JENNINGS & EVERY 53-64 Chancery
Lane
London WC2A 1HN(GB)**

EP 0 181 342 B1

Chemical Abstracts, vol. 90, 1979, Shukla, species variation in manganese induced changes in brain biogenic amines, abst. no. 198507v

Chemical Abstracts, vol. 98, 1983, Kaliman, Biogenic monoamines and their precursors in rats with spontaneous arterial hypertension, abstract no. 195890u

Chemical Abstracts, vol. 99, 1983, Chandra, Psychiatric illness due to manganese poisoning, abstract. no. 100473u

The American Journal of Clinical Nutrition, vol. 6, no. 5, 1958, Salmon, The significance of amino acid balance in nutrition, pages 487-494

## Description

BACKGROUND OF THE INVENTION

1. Field of the invention

Essential hypertension is one of the most general of the diseases occurring in western civilization. It is commonly associated with changes in lipid metabolism and atheromatosis. The changes in atherosclerosis produce the principal causes of arterial occlusion and death in hypertensives, e.g., coronary heart disease, cerebro-vascular accidents, renal insufficiency and intermittent claudication.

The chemical changes that occur in hypertension in humans occur in other species. The level of blood pressure directly reflects changes in the metabolism of biogenic amines. The increased pressures encountered result from a relative preponderance of catecholamines, especially norepinephrine. The oxidative processes involved in the modulation of catecholamine levels and the levels of other biogenic amines are directly involved in maintaining the blood pressure within normal limits.

Toxemia of pregnancy is characterized by hypertension occurring during pregnancy. The pathological changes occurring in the placenta and fetus directly relate to the vasoconstriction occurring as a result of the hypertension.

In addition to vasoconstriction and hypertension, fluid retention is a third feature of toxemia of pregnancy that is constantly present.

The medical use of tryptophan and manganese compounds is known, e.g. as described in US-A-4167564 and GB-A-1356370 (LU-A-0067165).

Summary of the Invention

According to the present invention, (a) an amino-acid selected from L- or D-tryptophan, and $\alpha$-keto, $\alpha$-hydroxy and acetyl analogues thereof, or a dipeptide or tripeptide form thereof, or a pharmaceutically-acceptable acid addition salt thereof, and (b) a manganese compound, are used for treating hypertension in vertebrates, or for treating toxemia of pregnancy in Placentalia.

The present invention is based on the utility of manganese-containing pharmaceutical preparations in decelerating the rate of oxidation of biogenic amines. The resulting increased levels of amines cause higher levels of activity in most cells in the organism. Shifts in biological parameters such as blood pressure occur. By combining the use of the manganese with tryptophan, the precursor of serotonin, there occurs a lowering of blood pressure.

Detailed Description of the Invention

In order that the invention may be more easily understood, the following Example is given by way of illustration only, to show details of the formulation for use in the invention, and the clinical test results obtainable using such formulations.

In this method, hypertension is best evaluated initially by the clinician personally whenever any treatment is undertaken. This is dictated by the changes in dosages that sometimes occur very quickly. It is desirable that the patient should be taken off all drugs when beginning treatment. In order to evaluate the actual pressures being experiences, it is necessary to know if there are any drug effects involved.

Because of the cumulative nature of the medication, treatment should be begun with small amounts and worked up to maximum dose. Then, almost as quickly, over a period of days, it may be necessary to decrease the dosage again. The manganese may be considered to modulate the tryptophan in its action as a precursor.

Example #1

Patient S.S.
Postmenopausal
Long history of hypertension.
Overweight, moderately.
Active.
Treatment periods:
Ranged from one day to five weeks.
Treatment:
manganese content in mg in manganese gluconate. tryptophan in one hundred mg tablets.
Treatment period interval:
four month period during spring and summer.
Objective findings:
Blood pressure
    A. Systolic pressure: ranged from 122 to 160 mm. Hg pressure (mm. mercury)
    B. Diastolic pressure: ranged from 70 to 90 millimeters Hg
    C. Pulse pressure: Ranged from 42 to 70 mm Hg ( = difference between systolic and diastolic)
Pulse:
ranged from 64 to 78/minute.
Range of Medication:
Manganese mg 1 to 3, usually 2 mg
Tryptophan:
none to 150 mg, usually 50 to 100 mg medication given about two out of three 'blood pressure checks'.
Tryptophan:
range of 0.67 to 2 mg/kg body wt.
Manganese:
range of 0.014 mg to 0.04 mg/kg B.W.
Objective of treatment:

Maintain blood pressure within normal range (e.g., diastolic 90 or below) Maintain proper ratio of affective tone and blood pressure control Subjective findings:

I Affect (emotional tone): tended to deteriorate when blood pressure control maintained almost entirely with tryptophan. Restored by increased use of manganese.

II Patient's opinion of effect of treatment: tended to be more favorably evaluated when more of the manganese was given, although in such small amounts.

Summary:

Clinical response:

On a very limited intake of the two medications, frequently none being given when the blood pressure was checked, for a four month period satisfactory control of blood pressure.

The manganese was given about sixty per cent of the time, the tryptophan somewhat more.

The tryptophan ratio to manganese, Tr/mn ranged from 14/1 to 150/1 (mg)

## Claims

1. Use of (a) an amino-acid selected from L- or D-tryptophan, and $\alpha$-keto, $\alpha$-hydroxy and acetyl analogues thereof, or a dipeptide or tripeptide form thereof, or a pharmaceutically-acceptable acid addition salt thereof, and (b) a manganese compound, for the manufacture of a composition for treating hypertension in vertebrates.

2. Use of components (a) and (b) as defined in claim 1, for the manufacture of a composition for treating toxemia of pregnancy in Placentalia.

3. Use according to claim 1 or claim 2, wherein the manganese compound is manganese gluconate.

4. Use according to any preceding claim, wherein component (a) is L-tryptophan.

## Patentansprüche

1. Verwendung (a) einer Aminosäure, die von L- oder D-Tryptophan, und $\alpha$-Keto, $\alpha$-Hydroxy und Acetalanalogonen davon ausgewählt wird, oder einer Dipeptid- oder Tripeptidform davon, oder einem pharmazeutisch akzeptierbaren säurehaltigen Salz davon, und (b) einer Manganverbindung zur Herstellung einer Zusammensetzung zur Behandlung von hohem Blutdruck in Wirbeltieren.

2. Verwendung der wie in Anspruch 1 definierten Komponenten (a) und (b) zur Herstellung einer Zusammensetzung zur Behandlung von Schwangerschaftstoxikose in placentaren Säugetieren.

3. Verwendung nach Anspruch 1 oder 2, in der die Manganverbindung Mangangluconat ist.

4. Verwendung nach einem der vorhergehenden Ansprüche, in der die Komponente (a) L-Tryptophan ist.

## Revendications

1. Exploitation (a) d'un amino-acide sélectionné à partir de tryptophane L ou D et de leurs analogues $\alpha$-keto, $\alpha$-hydroxy et acétyle, ou de sa forme dipeptide ou tripeptide, ou d'un sel additif acide admis en pratique pharmaceutique et (b) d'un composé de manganèse dans l'élaboration d'un composé pour le traitement de l'hypertension chez les vertébrés.

2. Exploitation des éléments (a) et (b) tels que revendiqués à la revendication 1, dans l'élaboration d'un composé pour le traitement de la toxémie de grossesse des placentaires.

3. Exploitation selon les revendications 1 ou 2, dont le composé de manganèse est le gluconate de manganèse.

4. Exploitation selon l'une quelconque des revendications précédentes, dont l'élément (a) est le tryptophane L.